(19) Europäisches Patentamt
European Patent Office
Office européen des brevets

(11) Veröffentlichungsnummer: **0 536 603 A1**

## EUROPÄISCHE PATENTANMELDUNG

(21) Anmeldenummer: **92116434.9**

(22) Anmeldetag: **25.09.92**

(51) Int. Cl.5: **C07D 211/90, A61K 31/44**

(30) Priorität: **08.10.91 DE 4133257**

(43) Veröffentlichungstag der Anmeldung:
**14.04.93 Patentblatt 93/15**

(84) Benannte Vertragsstaaten:
**AT BE CH DE DK ES FR GB GR IE IT LI LU MC NL PT SE**

(71) Anmelder: **BAYER AG**

**W-5090 Leverkusen 1 Bayerwerk(DE)**

(72) Erfinder: **Meier, Heinrich, Dr.**
**Briller Strasse 99**
**W-5600 Wuppertal 1(DE)**
Erfinder: **Meyer, Horst, Prof. Dr.**
**Henselweg 11**
**W-5600 Wuppertal 1(DE)**
Erfinder: **Wehinger, Egbert, Dr.**
**Gellertweg 33**
**W-5600 Wuppertal 1(DE)**
Erfinder: **de Jonge, Marten, Dr.**
**Am Kreuzberg 9**
**W-5063 Overath-Steinenbrück(DE)**
Erfinder: **Opitz, Wolfgang, Dr.**
**Holzbachtalstrasse 60**
**W-5063 Overath(DE)**

(54) **N-Methyl-nimodipin, Verfahren zu seiner Herstellung und seine Verwendung als Zerebraltherapeutikum.**

(57) Die Erfindung betrifft die neue Verbindung 1,2,6-Trimethyl-4-(3-nitrophenyl)-1,4-dihydropyridin-3,5-dicarbonsäure-isopropyl-2-methoxyethylester, als Racemat, (+)-Enantiomer und als (-)-Enantiomer, Verfahren zu ihrer Herstellung sowie ihre Verwendung als Arzneimittel, insbesondere als zerebral wirksames Mittel.

EP 0 536 603 A1

Die Erfindung betrifft die neue Verbindung 1,2,6-Trimethyl-4-(3-nitrophenyl)-1,4-dihydropyridin-3,5-dicarbonsäure-isopropyl-2-methoxyethylester als Racemat, als (+)-Enantiomer und als (-)-Enantiomer (nachfolgend N-Methyl-nimodipin genannt), Verfahren zu seiner Herstellung sowie seine Verwendung als Arzneimittel, insbesondere als zerebral wirksames Mittel.

N-Alkyl-dihydropyridine und Verfahren zu ihrer Herstellung sind bereits bekannt (vgl. DOS 19 23 990 und DOS 18 13 436). Die Verbindung Nimodipin und ihre zerebrale Wirksamkeit sind ebenfalls bekannt (vgl. DOS 28 15 578). Bisherige Untersuchungen von 1,4-Dihydropyridinen, die in 1-Position durch Alkyl substituiert sind, haben immer eine signifikant schwächere Wirkung im Vergleich zu den unsubstituierten Hydropyridinen gezeigt.

Überraschenderweise zeigt sich, daß nach oraler Applikation von N-Methyl-nimodipin bereits nach 15 Minuten Plasmaspiegel von Nimodipin und bekannten Nimodipinmetaboliten erreicht werden, die mindestens so hoch sind wie die entsprechenden Plasmaspiegel nach Mehrfachapplikation entsprechender Nimodipinmengen. Zum gleichen Zeitpunkt nach Applikation findet man überraschenderweise eine etwa doppelt so hohe Plasmakonzentration an unverändertem N-Methyl-Nimodipin, das erst in der Folge zu Nimodipin metabolisiert wird.

Dies bedeutet, daß durch Verabreichung des erfindungsgemäßen N-Methyl-Nimodipin als Prodrug eine Verbesserung der pharmakokinetischen Parameter Bioverfügbarkeit und Plasmahalbwertszeit für den Wirkstoff Nimodipin erzielt wird, d.h. eine bessere Wirkstoffausnutzung und eine Verlängerung der Wirkung.

Die vorzügliche Eignung von N-Methyl-Nimodipin als Prodrug für Nimodipin zeigt sich auch in seiner antidepressiven Wirkung. Im Rattenschwimmtest (vgl. R.D. Porsolt et al., European Journal of Pharmacology 47; 379 bis 391) zeigen Ratten, die 20 mg der erfindungsgemäßen Verbindung per os appliziert bekommen, eine um ca. 20 % reduzierte Immobilitätsphase im Vergleich zur Kontrolle (n = 6). Nach Applikation einer entsprechenden Menge von Nimodipin erhält man vergleichbare Ergebnisse in diesem Test.

Die Herstellung der erfindungsgemäßen Verbindungen erfolgt nach üblichen Methoden, z.B. durch Methylierung von Nimodipin oder seinen Enantiomeren oder durch direkte Cyclisierung entsprechender Alkylaminverbindungen gemäß DOS 19 23 990. Bevorzugt ist die Methylierung des Nimodipins oder seiner Enantiomeren in Gegenwart von wasserfreien organischen Lösungsmitteln bei Temperaturen zwischen -10 und +20°C durch Umsetzung mit Iodmethan in Gegenwart von starken Basen wie Natriumhydrid.

Die erfindungsgemäße Verbindung kann nach üblichen Methoden in galenische Zubereitungen überführt werden. Sie ist besonders bevorzugt für die Herstellung von Retardzubereitungen.

Herstellungsbeispiel

(±)-1,2,6-Trimethyl-4-(3-nitrophenyl)-1,4-dihydropyridin-3,5-dicarbonsäure-isopropyl-2-methoxyethyl-ester (N-Methyl-nimodipin)

Zu einer gerührten Lösung von 10,0 g (23,9 mmol) (±)2,6-Dimethyl-4-(3-nitrophenyl)-1,4-dihydropyridin-3,5-dicarbonsäure-isopropyl-2-methoxyethyl-ester (Nimodipin) in 125 ml wasserfreiem 1,2-Dimethoxyethan gibt man bei 0°C unter Argon portionsweise 0,70 g (ca. 23 mmol) Natriumhydrid (ca. 80 %ig in Weißöl) und rührt für 30 Minuten bei 0°C. Dann gibt man 1,8 ml (29 mmol) Iodmethan zu, rührt 1 h bei 0°C und 1,5 h bei Zimmertemperatur. Man filtriert, stellt mit Eisessig sauer und engt im Vakuum ein. Der Rückstand wird in Essigester aufgenommen und zweimal mit Wasser gewaschen. Der nach Trocknen und erneutem Einengen verbleibende Rückstand wird durch Säulenfiltration über 300 ml Kieselgel in Toluol/Essigester (Essigesteranteil stufenweise von ca. 1 % auf 10 % steigernd) gereinigt.

Man erhält so 9,9 g der Zielverbindung als gelbes Öl, das allmählich kristallisiert. Verrühren mit Diethylether/Petrolether 1:1 und Absaugen der erhaltenen Kristalle ergibt 7,5 g (73 % der Theorie) der Zielverbindung als weiße Kristalle, Schmelzpunkt ca. 70 bis 75°C (Zers.).

In analoger Weise wurde erhalten aus (+)-2,6-Dimethyl-4-(3-nitrophenyl)-1,4-dihydropyridin-3,5-dicarbonsäureisopropyl-2-methoxyethyl-ester der entsprechende (+)-1,2,6-Trimethyl-dihydropyridinester ((+)-N-Methylnimodipin) als gelbes Öl in einer Ausbeute von 67 %.

$R_f$ (SiO$_2$, Toluol/Ethylacetat 1:1): 0,66

$\alpha_D^{20}$ = +30 (CHCl$_3$; c = 0,76)

und

aus dem entsprechenden (-)-2,6-Dimethyl-dihydropyridinester der (-)-1,2,6-Trimethyl-dihydropyridinester ((-)-N-Methyl-nimodipin) als gelbes Öl mit einer Ausbeute von 68 %.

$R_f$ (SiO$_2$, Toluol/Ethylacetat 1:1): 0,66

$\alpha_D^{20}$ = -29 (CHCl$_3$; c = 1,24).

**Patentansprüche**

1. 1,2,6-Trimethyl-4-(3-nitrophenyl)-1,4-dihydropyridin-3,5-dicarbonsäure-isopropyl-2-methoxyethylester als Racemat, (+)-Enantiomer und (-)-Enantiomer.

2.  Verwendung der Verbindungen gemäß Anspruch 1 bei der Herstellung von zerebral wirksamen Arzneimitteln.

3.  Verfahren zur Herstellung der Verbindungen gemäß Anspruch 1, dadurch gekennzeichnet, daß man 2,6-Dimethyl-4-(3-nitrophenyl)-1,4-dihydropyridin-3,5-dicarbonsäure-isopropyl-2-methoxyethylester in inerten organischen Lösungsmitteln bei Temperaturen zwischen -10°C und +20°C gegebenenfalls in Gegenwart von Natriumhydrid mit Iodmethan methyliert und anschließend nach üblichen Methoden abtrennt und reinigt.

4.  Arzneimittel enthaltend eine Verbindung gemäß Anspruch 1.

5.  Arzneimittel in Retardform zur Behandlung von cerebralen Erkrankungen enthaltend eine Verbindung gemäß Anspruch 1.

6.  Verfahren zur Herstellung von Arzneimitteln, dadurch gekennzeichnet, daß man eine Verbindung gemäß Anspruch 1 gegebenenfalls unter Verwendung von üblichen Hilfs- und Trägerstoffen in eine geeignete Applikationsform überführt.

Europäisches
Patentamt

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| P,Y | EP-A-0 451 654 (BAYER AG) 16. Oktober 1991<br>* das ganze Dokument *<br>--- | 1-6 | C07D211/90<br>A61K31/44 |
| Y<br><br>D | EP-A-0 004 650 (BAYER AG) 17. Oktober 1979<br>* das ganze Dokument *<br>& DE-A-2 815 578<br>--- | 1-6 | |
| Y | DE-A-2 210 672 (BAYER AG) 20. September 1973<br>* Seite 16; Ansprüche 1-4; Beispiel 2 *<br>* Seite 11; die 4. Verbindung *<br>--- | 1-6 | |
| D,Y | DE-A-1 813 436 (FARBENFABRIKEN BAYER AG) 29. Oktober 1970<br>* Seite 7; Beispiel 2 *<br>* Seite 2, vorletzter Absatz *<br>--- | 1-6 | |
| 0,A | DE-A-1 923 990 (FARBENFABRIKEN BAYER AG) 19. November 1970<br>* das ganze Dokument *<br>* insbesondere siehe Seite 4, letzter Absatz *<br><br>----- | 1-6 | |

| | RECHERCHIERTE SACHGEBIETE (Int. Cl.5 ) |
|---|---|
| | C07D<br>A61K |

Der vorliegende Recherchenbericht wurde für alle Patentansprüche erstellt

| Recherchenort | Abschlußdatum der Recherche | Prüfer |
|---|---|---|
| MUENCHEN | 03 DEZEMBER 1992 | HARTRAMPF G.W. |